# EUROPEAN PATENT APPLICATION

(11) **EP 0 544 472 A1**
(43) Date of publication of application: **02.06.1993**
(21) Application number: 92310632.2
(22) Date of filing: 20.11.1992
(51) Int. Cl.: A01H 1/04

(54) **Method of screening for disease or pest resistance amongst crop plant varieties**

(30) Priority: 21.11.1991 GB 9124751
(71) Applicant: KNIGHTS, Christopher Robin, King's Lynn, Norfolk PE33 9DA (GB)
(72) Inventor: Groom, Murree, Leziate, King's Lynn, Norfolk (GB)
(74) Representative: Jones, William

(57) **Abstract**

A method of screening for resistance to a particular disease or pest amongst one or more varieties, or strains, of a particular crop plant which comprises assessing the uptake by each strain under test of a selected marker compound and comparing the uptake of that marker compound by the test strain with the corresponding uptake of the marker compound by a reference strain, or strains, of the same crop plant which has been treated under closely similar conditions and of which reference strain(s) the level of resistance to the disease or pest is/are known.

## Description

### Field of the Invention

The present invention relates to a method of screening for resistance to disease or pest damage amongst crop plant varieties, and more particularly, but not exclusively, amongst carrot or parsnip varieties.

### Background to the Invention

The concept of disease resistance in plants is now widely appreciated and both academic and commercial institutions apply very substantial resources in the study of plant characteristics for commercial gain.

With reference to carrots, one of the main pathogens of this crop is Pythium violae, a fungus that causes the disease known as cavity spot, and there are now numerous reports from around the world linking the disease to this fungus and occasionally to other Pythium species. Recently (since 1985) there has been some debate as to whether cavity spot incidence is influenced by the variety of carrot, and, if so, whether characteristics of disease resistance can be bred.

The conventional approach to screening for disease resistance of strains of a particular crop plant in plant breeding programmes involves a direct biological screening protocol in which the actual incidence of disease in each new variety under test is assessed through the cultivation of the variety in association with the pathogen over a period of time. This widely used approach is subject to very high inherent variability in the results obtained and high expenditure of both time and resources. Many screening programmes last for in excess of ten years. Consequently, any approach to disease resistance screening which reduces the variability of the results or the time involved in the screening operation should have major financial advantages and lead to higher confidence in the disease resistance characteristics of the finally selected strain of crop plant.

### Summary of the Invention

According to the present invention there is provided a method of screening for resistance to a particular disease or pest amongst one or more varieties, or strains, of a particular crop plant which comprises assessing the uptake by each strain under test of a selected marker compound and comparing the uptake of that marker compound by the test strain with the corresponding uptake of the marker compound by a reference strain or strains of the same crop plant which has been treated under closely similar conditions and of which reference strain(s) the level of resistance to the disease or pest is/are known.

This screening protocol greatly accelerates the rate at which new strains of a particular crop plant may be assessed for resistance to disease or pest damage, reducing the overall time scale from a number of years to a few months.

The protocol suitably involves cultivating each strain under test to a selected stage of growth prior to applying the marker compound, which is suitably an agrochemical such as a pesticide, and subsequently taking one or more samples from each strain and analysing the tissue of each sample for its content of the marker. Preferably a sample is taken at time of application of the marker to the crop and the marker content of the sample is assessed and a corresponding further sample is taken several days after application of the marker.

Preferably the reference strain or strains are cultivated alongside the test strains to be subjected to closely similar conditions and to enable the uptake of the marker by the reference strain(s) to be assessed for direct comparison with the test strain assessment.

Suitably several reference strains are cultivated alongside the test strains to enable a standard curve to be plotted relating disease resistance level to observed uptake of the marker under the test conditions. A quantitative assessment of the disease/pest damage resistance of each test strain may then be obtained by reading from the plotted standard curve the resistance level which corresponds to the observed uptake of marker by the test strain, or by calculating the resistance level using the equation of the standard curve.

Where the method is applied as a tool for screening for resistance of carrot strains to cavity spot disease a metalaxyl-based fungicide is an especially preferred marker compound.

### Brief Description of the Drawing

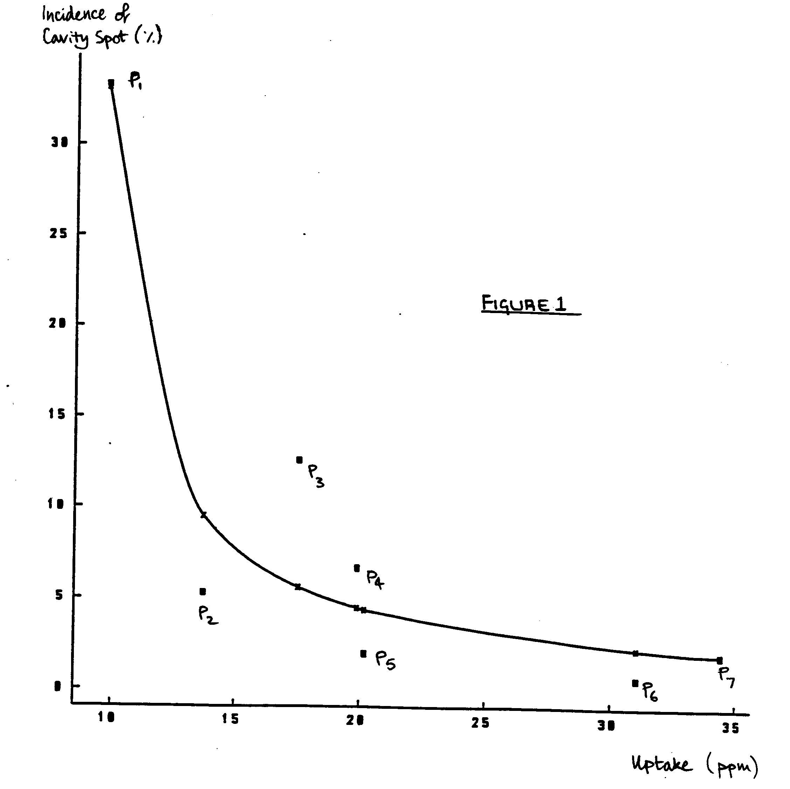

Figure 1 is a regression curve illustrating the correlation of incidence of cavity spot disease amongst an untreated range of strains of carrot relative to the corresponding uptake of metalaxyl by metalaxyl-treated samples of each strain, the treated and untreated samples having been grown alongside each other.

### Description of the Preferred Embodiments

During extensive experimental trials which were originally set-up to determine the efficacy of a new type of pesticide applicator it was discovered that the level of pesticide residues in the tissues of a root crop plant after a period of application of the pesticide to that plant bears close correlation to the ability of that plant to resist the pest or disease whether treated or not treated with the pesticide and, importantly, observed varietal differences in the pesticide residue content correlate to the disease or pest damage resistance characteristic of each variety.

In one series of trials application of metalaxyl, in the form of FUBOL (rtm), to a range of different strains of carrot under closely similar experimental conditions gave rise, after a period of time, to levels of residue of metalaxyl in the carrots which differed between the strains and correlated to the known cavity spot disease resistance characteristics of the respective strains.

Using a reliable applicator such as the combined cultivator and fluid injector system described in GB-A-9118368.1, fixed doses of metalaxyl are applied to a crop of a test strain of carrots in a controlled fashion with a controlled dosage and at a pre-determined stage in the growth of the crop with samples then being taken from the crop after a fixed period of a few days post-application to assess the residues of metalaxyl in the tissue of the carrot. A suitable stage for application of the FUBOL to the carrot crop is at ninety days post sowing or, most usefully, when the roots of the plants are clearly defined and the sixth true leaf has formed. Samples of the crop may then be analysed for residue, following standard procedures and thoroughly washing the samples, after a period of, for example, four to seven days following pesticide application. As a reference marker, a carrot variety of known disease resistance is suitably grown physically and temporally alongside the variety of unknown resistance and treated in the same manner.

Samples of both the test and reference crop strains are suitably taken at the time of application of the pesticide to serve as controls to enable the true tissue uptake of the pesticide to be assessed, for example removing the variability induced by the superficial deposits of pesticide on the exterior of the plants. The samples taken at time of pesticide application and seven days post application are independently homogenised, weighed and then subjected to an extraction process for the pesticide. A specific portion of the plant may be chosen for assessment.

To extract metalaxyl, which is an acyl alanine compound, acetonitrile solution is used, suitably containing an internal standard of dibutyl phthalate. The extracts are then subjected to analysis for metalaxyl content by gas chromatography.

The uptake of the pesticide by each strain of crop is determined as the concentration (in parts per million) of the pesticide in the post-application sample minus the corresponding concentration in the sample taken at the time of application of the pesticide.

Using the conventional indicator of cavity spot disease resistance of a strain, the number of spots per carrot of fixed size or percentage area of sample covered by spots, a graph or equation indicating the correlation between cavity spot resistance of a range of different strains and their corresponding metalaxyl uptake under closely similar controlled conditions may be derived.

Referring to Figure 1 there is shown a regression curve for the correlation between susceptibility to cavity spot disease of a range of different carrot strains which have not been treated with metalaxyl and the affinity for uptake of metalaxyl by the same strains treated with metalaxyl but grown and sampled under closely similar conditions. The results show a marked difference in uptake of metalaxyl between carrot strains. Furthermore, there is clear indication that high affinity for metalaxyl corresponds to high natural disease resistance in a particular strain, surprisingly even without treatment of the strain with the pesticide. The auxiliary effect of high metalaxyl affinity of a given strain on the ability of that strain to resist cavity spot disease is, therefore, not the sole cause of the varietal metalaxyl affinity/cavity spot resistance correlation.

The plots, P1 to P7 on the graph correspond to strains Nanco, Nandor, Liberno, Major, Fancy, Nairobi and Narman, respectively.

The highly significant differences (variance ratio 99.99, confidence limit 99.9) in metalaxyl uptake/accumulation between different carrot strains of extremely differing disease resistance are highlighted by the fact that the popular comparatively high disease resistance strain Nairobi demonstrated a metalaxyl uptake approximately three and a half times greater than that of Nanco an unpopular comparatively poorly disease resistant strain when the two strains were subjected to a comparative trial under substantially identical experimental conditions.

Further trials with different crops and marker compounds are underway and have shown that the method of the invention has broad applicability. For example, trials have been carried out with Triazaphos, an insecticide active against carrot fly, and demonstrated that strains of carrot such as "Fancy" with a high affinity for Triazaphos, are less susceptible to insect damage than low affinity strains such as "Nandor".

Routine trials can be performed to identify other suitable marker compounds for different diseases or crops. Ideally the marker is an agrochemical which is harmless to the plants being screened.

As an alternative to growing the reference crop strain(s) alongside the test strain(s), extensive tables of results of earlier assessments of uptake of the marker by each of a range of reference strains for each of a range of relevant environmental conditions may be drawn up to be referred to in individual trials of test strains. Environmental conditions of relevance for which results should be made available may include, for example, soil pH and bulk density and extent of soil water-logging at the time of the trial.

## Claims

1. A method of screening for resistance to a particular disease or pest amongst one or more varieties, or strains, of a particular crop plant which comprises assessing the uptake by each strain under test of a selected marker compound and comparing the uptake of that marker compound by the test strain with the corresponding uptake of the marker compound by a reference strain, or strains, of the same crop plant which has been treated under closely similar conditions and of which reference strain(s) the level of resistance to the disease or pest is/are known.

2. A method as claimed in Claim 1, wherein each strain under test is grown to a selected stage of growth prior to applying the marker compound and subsequently taking one or more samples from each strain and analysing the tissue of each sample for its content of the marker.

3. A method as claimed in Claim 2, wherein a sample is taken at time of application of the marker to the crop and the marker content of the sample is assessed and a corresponding further sample is taken several days after application of the marker to the crop.

4. A method as claimed in any preceding Claim, wherein the reference strain, or strains, are cultivated alongside the test strain(s) to be subjected to closely similar conditions for direct comparison for marker uptake with the test strain.

5. A method as claimed in Claim 4, wherein several reference strains having a range of different resistance levels to the disease are cultivated alongside the test strains to enable a standard curve to be plotted relating disease resistance level to observed uptake of the marker under the test conditions.

6. A method as claimed in any preceding Claim, where in the marker compound is an agrochemical which is not harmful to the crop plant.

7. A method as claimed in any preceding Claim, wherein the crop plant is carrot.

8. A method as claimed in Claim 7, wherein the disease is cavity spot disease and the marker compound is a metalaxyl-based fungicide.
